# EUROPÄISCHE PATENTSCHRIFT

(11) **EP 0 900 814 B1**
(45) Veröffentlichungstag und Bekanntmachung des Hinweises auf die Patenterteilung: **21.12.2005**
(21) Anmeldenummer: 98115771.2
(22) Anmeldetag: 21.08.1998
(51) Int. Cl.: C08G 18/80

(54) **Amin-blockierte Polyisocyanate**
Amine-blocked polyisocyanates
Polyisocyanates bloqués par des amines

(30) Priorität: 03.09.1997 DE 19738497
(43) Veröffentlichungstag der Anmeldung: 10.03.1999
(73) Patentinhaber: Bayer MaterialScience AG, 51368 Leverkusen (DE)
(72) Erfinder: König, Eberhard, Dr., 51375 Leverkusen (DE); Noble, Karl-Ludwig, Dr., 51467 Bergisch Gladbach (DE); Füssel, Christian, 47918 Tönisvorst (DE)

(56) Entgegenhaltungen:
- EP-A- 0 178 398
- EP-A- 0 654 490
- EP-A- 0 741 157

## Beschreibung

Die Erfindung betrifft nicht kristallisierende, aminblockierte Polyisocyanate, deren Herstellung und Verwendung in 1K-PUR-Einbrennlacken, insbesondere für Coil-Coating-Anwendungen.

Es gibt nur wenige Isocyanat-Blockierungsmittel, die einerseits über gute chemische Eigenschaften, z.B. geringe Thermovergilbung und eine Deblockierungsneigung bei relativ niedriger Temperatur verfügen, andererseits preiswert zur Verfügung stehen. Diisopropylamin ist ein solches Blockierungsmittel.

Die Verwendung von Diisopropylamin als Isocyanat-Blockierungsmittel gehört zum Stand der Technik. So wird beispielsweise in derEP-96 210/Hüls die Herstellung und Verwendung eines mit Diisopropylamin blockierten Polyisocyanates, basierend auf trimerisiertem Isophorondiisocyanat, beschrieben.

In der EP-A-600 314 wird der Einsatz von gemischten Blockierungsmitteln, z.B. Mischblockierungen aus Diisopropylamin und Malonester, aufgezeigt. Hier wird aber auch auf einen Nachteil von Diisopropylamin hingewiesen, nämlich mit bestimmten Polyisocyanaten keine lagerstabilen blockierten Polyisocyanate zu bilden, sondern in gelöster Form auszukristallisieren.

Ein technisch und wirtschaftlich wichtiges Polyisocyanat stellt das Isocyanuratgruppen aufweisende Polyisocyanat auf Basis von 1,6-Diisocyanatohexan dar, z.B. trimerisiertes HDI. Dieses Polyisocyanat verleiht bekanntermaßen den PUR-Lacken ein besonders gutes zäh-elastisches Verhalten. Es macht die Lacke biegbar und kratzfest.

Coil- und Can-Coating-Lacke benötigten ein besonders gutes Flexibilitätsverhalten, weil diese Lacke die spätere Verformung der lackierten Bleche ohne Beschädigung mitmachen müssen.

In der EP-A-600 314 wird darauf hingewiesen, daß trimerisiertes HDI z.B. mit Diisopropylamin nicht zu einem lagerstabilen Vernetzer für gelöste 1K-PUR-Einbrennlacke verarbeitet werden kann, weil so blockierte Polyisocyanate aus der Lösung auskristallisieren.

Die der Erfindung zugrundeliegende Aufgabe bestand darin, mit speziellen sekundären Aminen als Blockierungsmittel und Polyisocyanaten einen lagerstabilen, in Lösung nicht kristallisierenden Polyisocyanat-Vernetzer mit geringer Thermovergilbung für 1K-PUR-Einbrennlacke, insbesondere für das Coil-Coating-Verfahren, zur Verfügung zu stellen.

Gegenstand der Erfindung sind lagerstabile, in Lösung nicht kristallisierende blockierte Polyisocyanat-Vernetzer, die Umsetzungsprodukte darstellen von
A1) 30 bis 70 Äquivalent-% einer Polyisocyanatkomponente mit einem NCO-Gehalt von 12 bis 24 Gew.-%, bestehend aus Allophanat-, Biuret-, Isocyanurat-, Uretdion- und/oder Urethangruppen aufweisenden Lackpolyisocyanaten auf Basis von linaren, gegebenenfalls verzweigten aliphatischen Diisocyanaten und
A2) 30 bis 70 Äquivalent-% einer Polyisocyanatkomponente mit einem NCO-Gehalt von 8 bis 15 Gew.-%, bestehend aus Allophanat-, Biuret-, Isocyanurat-, Uretdion- und/oder Urethangruppen aufweisenden Lackpolyisocyanaten auf Basis von cycloaliphatischen Diisocyanaten, wobei sich die beiden Polyisocyanatkomponenten A1 + A2 zu 100 Äquivalent-% addieren, mit
B) 85 bis 100 Äquivalent-%, bezogen auf die Isocyanatgruppen der Komponenten A1 + A2, eines sekundären aliphatischen linearen Amins mit einem Siedepunkt von 60 bis 110°C und
C) bis 15 Äquivalent-%, bezogen auf die Isocyanatgruppen der Komponenten A1 + A2, an einbaubaren Hydrazinderivaten der Formel (I) 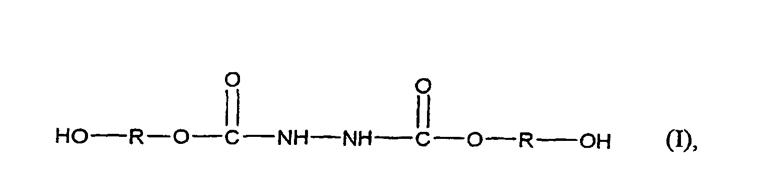 in welcher
   - R: für einen gesättigten Kohlenwasserstoffrest mit 2 bis 5 Kohlenstoffatomen steht,
   und die zugemischt enthalten
D) bis 5,0 Gew.-%, bezogen auf die Komponenten A1, A2 und C, Amine mit der Struktureinheit der Formel (II)

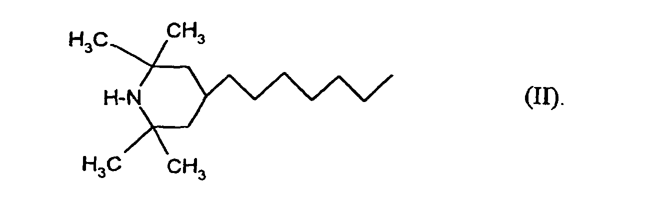

Gegenstand der Erfindung ist auch ein Verfahren zur Herstellung dieser blockierten Polyisocyanate in Lacklösemitteln, dadurch gekennzeichnet, daß man die Polyisocyanatkomponenten A1 + A2 in einem Teil des Gesamtlösemittels vorlegt, mit dem sekundären aliphatischen Amin mit einem Siedepunkt von 60 bis 110°C B) und gegebenenfalls anschließend mit der Stabilisatorkomponente C) bis zum Verbrauch der vorhandenen NCO-Gruppen umsetzt und daß man anschließend die Stabilisatorkomponente D) zusammen mit dem Restlösemittel hinzumischt.

Gegenstand der Erfindung ist schließlich die Verwendung dieser blockierten Polyisocyanate als Vernetzer in Einkomponenten-PUR-Einbrennlacken, insbesondere für das Coil Coating-Verfahren sowie mit unter Verwendung dieser Vernetzer erhältlichen-PUR-Einbrennlacken im Coil-Coating-Verfahren einbrennlackierte Substrate.

Erfindungswesentlich für die neuen blockierten Polyisocyanate ist zum einen die gemischte Polyisocyanatkomponente (A1 + A2) und zum anderen der Einbau bzw. die Abmischung mit den Stabilisatoren C und D.

Als Polyisocyanatkomponenten A1 kommen die an sich bekannten Biuret-, Isocyanurat-, Iminooxadiazindion-, Allophanat- und/oder Uretdiongruppen aufweisenden Lackpolyisocyanate auf Basis von 1,6-Diisocyanatohexan mit einem NCO-Gehalt von 19 bis 25 Gew.-% in Betracht. Bevorzugt sind die überwiegend Isocyanuratgruppen enthaltenden Lackpolyisocyanate auf Basis von 1,6-Diisocyanatohexan, kurz trimerisiertes HDI.

Trimerisiertes HDI wird in einer Menge von 0,3 bis 0,7 NCO-Äquivalenten, bezogen auf 1,0 NCO-Äquivalent der Gesamt-NCO-Komponente eingesetzt. Dies entspricht ca. einer Menge von 24 bis 67 Gew.-%, bezogen auf die Summe der Polyisocyanatkomponenten A1 + A2 von 100 Gew.-%.

Als Polyisocyanatkomponente A2 kommen die an sich bekannten Allophanat-, Biuret-, Isocyanurat- und/oder Urethangruppen aufweisenden Derivate von cycloaliphatischen Diisocyanaten, wie insbesondere 1-Isocyanato-3,3,5-trimethyl-5-isocyanatomethyl-cyclohexan (IPDI) und 4,4'-Diisocyanato-dicyclohexylmethan (HMDI) in Betracht. Bevorzugt sind die überwiegend Isocyanuratgruppen aufweisenden Lackpolyisocyanate auf Basis von IPDI und HMDI in einer Menge von 0,3 bis 0,7 NCO-Äquivalenten, bezogen auf 1,0 NCO-Äquivalent der Gesamt-NCO-Komponente (A1 + A2).

Prinzipiell geeignet als Polyisocyanatkomponente A1 + A2 sind ebenfalls gemischte Trimerisate von HDI und IPDI sowie HDI und HMDI in den genannten Äquivalentverhältnissen.

Als sekundäre aliphatische Amine mit einem Siedepunkt von 60 bis 110°C (bei Raumtemperatur und Normaldruck) kommen in Betracht z.B. Diisopropylamin.

Bei der mitzuverwendenden Stabilisatorkomponente C handelt es sich um Hydrazinderivate der bereits oben genannten Formel. Die Herstellung dieser Hydrazinderivate erfolgt durch Hydrazinhydrat mit cyclischen Carbonaten, beispielsweise mit Ethylen- oder Isopropylencarbonat entsprechend den in EP-A-0 050 284 diesbezüglich gemachten Ausführungen.

Bevorzugt wird das Addukt aus 1 mol Hydrazin und 2 mol Propylen-1,2-carbonat eingesetzt:

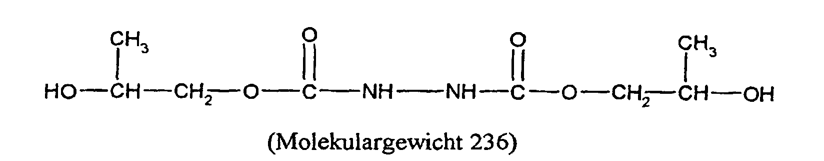

Als Stabilisierungsmittel D der Formel (I) können Verbindungen mit mindestens einem 2,2,6,6-Tetramethylpiperidinyl-Rest, dem sogenannten HALS (hindered amine light stabilizer)-Ring eingesetzt werden. Hervorzuheben ist, daß bei den eingesetzten HALS-Verbindungen der Piperidinyl-Stickstoff nicht substituiert, also gemäß folgender Struktur erhalten sein muß:

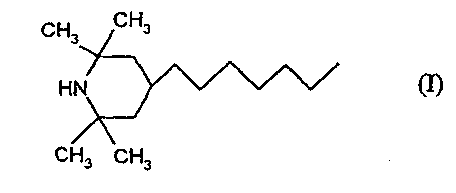

Ein besonders bevorzugtes Stabilisierungsmittel ist hierbei die u.a. von der Fa. Novartis unter der Bezeichnung Tinuvin 770 DF® vertriebene "HALS"-Verbindung der Formel (II)

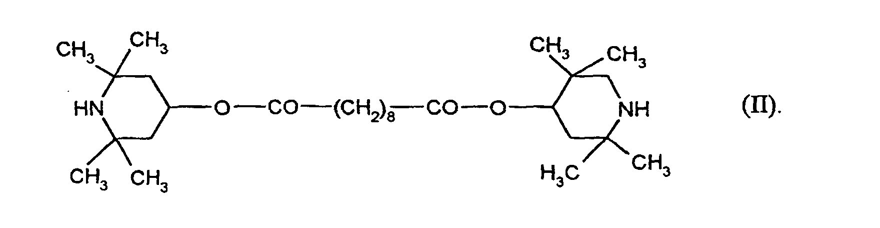

Die Durchführung des erfindungsgemäßen Verfahrens erfolgt im Temperaturbereich von 20 bis 120°C, vorzugsweise 70 bis 90°C, in geeigneten Lösemitteln wie beispielsweise n-Butylacetat, Methoxypropylacetat, Toluol oder höheren aromatischen Lösemittelgemischen, wie sie beispielsweise von der Firma Exxon-Chemie unter der Bezeichnung ®Solvesso vertrieben werden. Allerdings können auch Alkohole, z.B. Isobutanol, eingesetzt werden, sobald die NCO-Gruppen vollständig mit dem Blockierungsmittel, z.B. Diisopropylamin (B) und dem Stabilisator C abreagiert sind.

Beispielsweise kann das erfindungsgemäße Verfahren nach folgender Verfahrensvariante durchgeführt werden: Man legt 1,0 NCO-Äquivalent der Mischung aus den Polyisocyanatkomponenten A1 + A2 gelöst vor und gibt hierzu die Gesamtmenge an Diisopropylamin (B), z.B. 0,9 Äquivalente (0,9 mol) und läßt bei ca. 70°C abreagieren, was relativ schnell vor sich geht. Danach wird mit 0,1 bis 0,15 OH-Äquivalenten der obengenannten Hydrazid-Komponente C mehrere Stunden bei ca. 80°C umgesetzt, bis sämtliche NCO-Gruppen abreagiert sind. Ein geringer stöchiometrischer Überschuß an der Komponente C wirkt sich hierbei günstig auf eine niedrige Viskosität des Endproduktes aus. Anschließend mischt man die HALS-Komponente D und Restlösemittel, beispielsweise Isobutanol, hinzu und stellt damit auf die gewünschte Viskosität ein.

Die erfindungsgemäßen blockierten Polyisocyanate dienen in Kombination mit organischen Polyhydroxylverbindungen, beispielsweise den an sich bekannten Polyesterpolyolen, Polyacrylatpolyolen oder deren Gemischen, zur Herstellung von 1K-PUR-Einbrennlacken, insbesondere Coil Coating-Lacken.

Die mit den erfindungsgemäßen Polyisocyanaten hergestellten Coil Coating-Lacke besitzen in vorteilhafter Weise eine gute Lagerstabilität, d.h. keinen nennenswerten Viskositätsanstieg über mindestens 3 Monate, eine gute Reaktivität und eine sehr geringe Thermovergilbung.

### Beispiele

### Beispiel 1 (nicht erfindungsgemäß)

Dieses Beispiel beschreibt die Zusammensetzung eines blockierten Polyisocyanates ohne die Stabilisatorkomponenten C und D. Die Polyisocyanatkomponente A1 besteht hierbei zu ca. 65,6 Gew.-% aus einem HDI-Trimerisat, Polyisocyanatkomponente A2 zu ca. 34,4 Gew.-% aus einem IPDI-Trimerisat.

### Ansatz:

| | |
|---|---|
| 140,0 g (0,7 Val) | HDI-Trimerisat, NCO-Gehalt ca. 21 %, Gehalt an monomerem 1,6-Diisocyanatohexan ca. 0,2 %, Viskosität bei 23°C ca. 3000 mPas |
| 105,0 g (0,3 Val) | IPDI-Trimerisat, NCO-Gehalt ca. 12 %, 70 %ige Lösung in Solventnaphta |
| 106,0 g (1,05 Val) | Diisopropylamin |
| 70,0 g | Methoxypropylacetat |
| 70,5 g | Isobutanol |
| 491,5 g (1,0 Val) | blockiertes Polyisocyanat, Festkörper: Ber. 65 % blockierter NCO-Gehalt: Ber. 8,5 % |

### Durchführung:

Die beiden Lackpolyisocyanate werden mit Methoxypropylacetat vorgelegt und auf 50°C erwärmt. Unter Rühren gibt man hierzu portionsweise Diisopropylamin, wobei eine leichte Exothermie beobachtet wird. Nach beendeter Zugabe wird 30 Min. bei 70°C nachgerührt. Mittels IR-Spektroskopie kann man hierbei das Verschwinden der NCO-Gruppen überprüfen. Sobald keine NCO-Gruppen IR-spektroskopisch mehr nachweisbar sind, wird mit Isobutanol verdünnt und man läßt erkalten.

Man erhält eine klare, fast farblose Lösung mit einer Viskosität (23°C) von ca. 3600 mPas und einem blockierten NCO-Äquivalent von 491,5 g.

Dieses blockierte Polyisocyanat ist lagerstabil. Wie aus der EP-A-600 314 bekannt ist, kristallisiert hingegen mit Diisopropylamin blockiertes HDI-Trimerisat nach ca. 2 Wochen aus.

### Beispiel 2

Bei diesem erfindungsgemäßen blockierten Polyisocyanat werden HDI-Trimerisat (A1) und IPDI-Trimerisat (A2) im Gew.-Verhältnis von ca. 1:1 eingesetzt.

### Ansatz:

| | |
|---|---|
| 110,0 g (0,55 Val) | HDI-Trimerisat, NCO-Gehalt ca. 21 % |
| 157,5 g (0,45 Val) | IPDI-Trimerisat, 70 %ige Lösung in Solventnaphta, NCO-Gehalt ca. 12 % |
| 90,9 g (0,9 moll) | Diisopropylamin |
| 70,0 g | Methoxypropylacetat |
| 14,1 g (0,12 Val) | Hydrazin-Addukt aus 1 mol Hydrazinhydrat und 2 mol Propylencarbonat, Molekulargewicht 236 |
| 3,2 g | Tinuvin® 770 DF |
| 64,8 g | Methoxypropylacetat |
| 64,8 g | Isobutanol |
| 505,3 g (0,9 Val) | blockiertes Polyisocyanat, Festkörper: Ber. 65 % blockierter NCO-Gehalt: Ber. 7,48 % |

### Durchführung:

Die beiden obigen Lackpolyisocyanate werden in Methoxypropylacetat vorgelegt und bei 70°C mit Diisopropylamin ca. 1 Stunde lang gerührt, bis ein NCO-Gehalt von ca. 1 % gemessen wird, berechnet sind 0,99 %. Danach gibt man das Hydrazin-Addukt hinzu, erhöht auf 80°C und rührt ca. 10 Stunden bei dieser Temperatur, bis keine NCO-Gruppen mehr nachweisbar sind (IR-Spektrum). In die noch warme Lösung wird Tinuvin® 770 DF, gelöst in Isobutanol, eingerührt. Man läßt abkühlen und erhält eine klare blaß-gelbe Lösung mit einer Viskosität (23°C) von ca. 6000 mPas und einem blockierten NCO-Äquivalent von 561 g.

### Beispiel 3 (nicht erfindungsgemäß)

Es wird die Herstellung eines praxisnahen Coil Coating-Lackes mit dem erfindungsgemäßen Polyisocyanat aus Beispiel 2 beschrieben. Die Eigenschaften dieses Lackes werden mit einem analogen, aber mit einem butanonoximblockierten Vernetzer (Basis HDI-Trimerisat) hergestellten Lack verglichen.

### Zusammensetzung des Lackes:

| | |
|---|---|
| 1172 g (1,172 Val OH) | Alkylnol® 1665, 1 OH-Äquivalent = 1000 g, 65 %ige Lösung eines Hydroxylpolyesters, Bayer AG |
| 657 g (1,172 Val NCO) | blockiertes Polyisocyanat gemäß Beispiel 2, 65 %ig |
| 1200 g | Bayertitan R-KB-4 |
| 940 g | Solventnaphta 200 S |
| 150 g | Celluloseacetobutyrat CAB 531-1, 10 %ig, Krahn Chemie/Hamburg |
| 60 g | Acronal 4 F, 50 %ig, BASF AG |
| 4179 g | Lackansatz |
| | Auslaufzeit: 118 sec DIN 4 |

| **Lackeigenschaften:** | | |
|---|---|---|
| Glanz 20°/60° | obiger Lack (erfindungsgemäßes Polyisocyanat) 85 - 92 | Vergleich (butanonoximblockiertes HDI-Trimerisat) 65 - 72 |
| Weißwert | | |
| PMT¹⁾ 216°C | 91,7 | - |
| PMT 232°C | | 84,1 |
| PMT 254°C | 90,5 | 82,6 |

| Gelbwert | | |
|---|---|---|
| PMT 216°C | -1,0 | |
| PMT 232°C | | -0,4 |
| PMT 254°C | -0,8 | -0,8 |

| MEK-Wischtest | | |
|---|---|---|
| PMT 216°C | 100 x | |
| PMT 232°C | | 100 x |
| Impact-Test | 80 o.B. | 80 o.B. |
| Bleistifthärte | F | HB |
| Haftfestigkeit 6 mm | 0 | 0 |
| Tiefung, GT | | |

| t-bend-Test | | |
|---|---|---|
| Rißfrei | 1,0 T | 1,0 T |
| Haftung OK | 1,0 T | 0,5 T |

| Auslaufzeit | | |
|---|---|---|
| sofort | 118 | 118 |
| nach 3 d RT/50°C | 124/150 | 124/140 |
| nach 14 d RT/50°C | 156/154 | 142/145 |

| | | |
|---|---|---|
| ¹⁾PMT = peak metal temperature, Objekttemperatur | | |

Wie man sieht, hat der Lack mit dem erfindungsgemäßen Polyisocyanat-Vernetzer einen überlegenen Glanz und einen deutlich höheren Weißwert nach dem Einbrennvorgang. Außerdem ist er reaktiver als der Vergleichslack. Letzteres wird aus der niedrigeren PMT von 216°C, bei der beispielsweise Weißwert-, Gelbwert- und MEK-Wischtest-Messungen durchführbar sind, ersichtlich.

### Beispiel 4

Hier besteht das Polyisocyanat zu 0,6 Äquivalenten bzw. ca. 50 Gew.-% aus einem HDI-Trimerisat und 0,4 Äquivalenten bzw. 50 Gew.-% aus einem HMDI-Trimerisat.

### Ansatz:

| | |
|---|---|
| 100,0 g (0,5 Val) | HDI-Trimerisat, NCO-Gehalt ca. 21 % |
| 189,5 g (0,5 Val) | eines Polyisocyanates aus 85 Gew.-% HMDI-Trimerisat und 15 Gew.-% HDI-Trimerisat 75 %ig in Methoxypropylacetat, |
| | NCO-Gehalt: 11,1 Gew.-% |
| 90,0 g (0,9 Mol) | Diisopropylamin |
| 14,1 g (0,12 Val) | Hydrazin-Addukt gemäß Beispiel 2 |
| 3,5 g | Tinuvin® 770 DF |
| 93,2 g | Methoxypropylacetat |
| 93,2 g | Isobutanol |
| 584,4 g | blockiertes Polyisocyanat |
| | Festkörper: Ber. 60 % |
| | blockierter NCO-Gehalt: Ber. 6,5 % |

### Durchführung:

Die beiden Lackpolyisocyanate werden mit Methoxypropylacetat vorgelegt und, wie bereits in den Beispielen 1 und 2 beschrieben, mit Diisopropylamin bei 70°C umgesetzt, bis der berechnete NCO-Gehalt von 0,88 % geringfügig unterschritten ist.

Danach fügt man das Hydrazin-Addukt hinzu und rührt ca. 10 Stunden bei 80°C. Danach ist kein NCO-Gehalt mehr feststellbar (IR-Spektrum). Man rührt Tinuvin® 770 DF, gelöst in Isobutanol, ein und läßt erkalten.

Man erhält eine klare, blaß-gelbe Lösung mit einer Viskosität (23°C) von ca. 4500 mPas und einem blockierten NCO-Äquivalent von 646 g.

## Patentansprüche

1. Lagerstabile, in Lösung nicht kristallisierende blockierte Polyisocyanat-Vernetzer, die Umsetzungsprodukte darstellen von
A1) 30 bis 70 Äquivalent-% einer Polyisocyanatkomponente mit einem NCO-Gehalt von 12 bis 24 Gew.-%, bestehend aus Allophanat-, Biuret-, Isocyanurat-, Iminooxadiazindion-, Uretdion- und/oder Urethangruppen aufweisenden Lackpolyisocyanaten auf Basis von linaren, gegebenenfalls verzweigten aliphatischen Diisocyanaten und
A2) 30 bis 70 Äquivalent-% einer Polyisocyanatkomponente mit einem NCO-Gehalt von 8 bis 15 Gew.-% bestehend aus Allophanat-, Biuret-, Isocyanurat-, Uretdion- und/oder Urethangruppen aufweisenden Lackpolyisocyanaten auf Basis von cycloaliphatischen Diisocyanaten, wobei sich die beiden Polyisocyanatkomponenten A1 + A2 zu 100 Äquivalent-% addieren, mit
B) 85 bis 100 Äquivalent-%, bezogen auf die Isocyanatgruppen der Komponenten A1 + A2, eines sekundären aliphatischen linearen Amins mit einem Siedepunkt von 60 bis 110°C und
C) bis 15 Äquivalent-%, bezogen auf die Isocyanatgruppen der Komponenten A1 + A2, an einbaubaren Hydrazinderivaten der Formel (I) 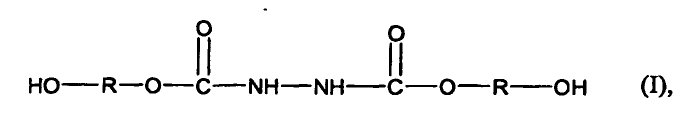 in welcher
R für einen gesättigten Kohlenwasserstoffrest mit 2 bis 5 Kohlenstoffatomen steht,
und die zugemischt enthalten
D) bis 5,0 Gew.-%, bezogen auf die Komponenten A1, A2 und C, Amine mit der Struktureinheit der Formel (II)

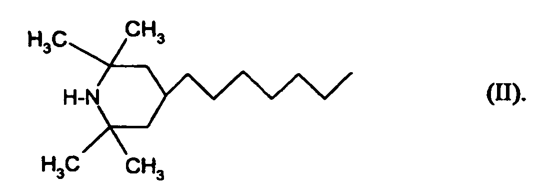

2. Blockierte Polyisocyanatvernetzer nach Anspruch 1, **dadurch gekennzeichnet, dass** Diisopropylamin als Komponente B) eingesetzt wird.

3. Verfahren zur Herstellung dieser blockierten Polyisocyanate in Lacklösemitteln, **dadurch gekennzeichnet, dass** man die Polyisocyanatkomponenten A1 + A2 in einem Teil des Gesamtlösemittels vorlegt, mit dem sekundären aliphatischen Amin mit einem Siedepunkt von 60 bis 110°C b) und gegebenenfalls anschließend mit der Stabilisatorkomponente C) bis zum Verbrauch der vorhandenen NCO-Gruppen umsetzt und dass man anschließend die Stabilisatorkomponente D) zusammen mit dem Restlösemittel hinzumischt.

4. Verwendung der blockierten Isocyanatvernetzer nach Anspruch 1 als Vernetzer in Einkomponenten-PUR-Einbrennlacken, insbesondere für das Coil Coating-Verfahren.

5. Mit unter Verwendung von blockierten Isocyanatvernetzern nach Ansprüchen 1 und 2 erhältlichen Einkomponenten-PUR-Einbrennlacken im Coil-Coating-Verfahren einbrennlackierte Substrate.

## Claims

1. Blocked polyisocyanate crosslinking agents that are stable in storage, do not crystallise in solution and constitute reaction products of
A1) 30 equivalent-% to 70 equivalent-% of a polyisocyanate component with an NCO content from 12 wt.% to 24 wt.%, consisting of lacquer polyisocyanates comprising allophanate, biuret, isocyanurate, iminooxadiazinedione, uretdione and/or urethane groups and based on linear, optionally branched, aliphatic diisocyanates and
A2) 30 equivalent-% to 70 equivalent-% of a polyisocyanate component with an NCO content from 8 wt.% to 15 wt.%, consisting of lacquer polyisocyanates comprising allophanate, biuret, isocyanurate, uretdione and/or urethane groups and based on cycloaliphatic diisocyanates, the two polyisocyanate components A1 + A2 adding up to 100 equivalent-%, with
B) 85 equivalent-% to 100 equivalent-%, relative to the isocyanate groups of components A1 + A2, of a secondary aliphatic linear amine having a boiling-point from 60 °C to 110 °C and
C) up to 15 equivalent-%, relative to the isocyanate groups of components A1 + A2, of incorporable hydrazine derivatives corresponding to Formula (I) 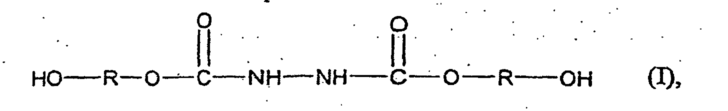 in which
R stands for a saturated hydrocarbon residue with 2 to 5 carbon atoms,
and which contain, admixed,
D) up to 5.0 wt.%, relative to components A1, A2 and C, amines having a structural unit corresponding to Formula (II)

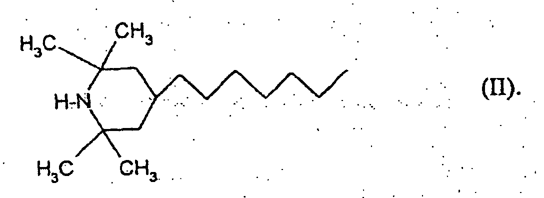

2. Blocked polyisocyanate crosslinking agents according to Claim 1, **characterised in that** diisopropylamine is employed as component B).

3. Process for preparing these blocked polyisocyanates in lacquer solvents, **characterised in that** the polyisocyanate components A1 + A2 are charged in a portion of the total solvent, caused to react with the secondary aliphatic amine B) having a boiling-point from 60 °C to 110 °C and, optionally, subsequently with the stabiliser component C) until the available NCO groups have been consumed and **in that** the stabiliser component D) is subsequently added, together with the residual solvent.

4. Use of the blocked isocyanate crosslinking agents according to Claim 1 as crosslinking agents in single-component polyurethane stoving lacquers, in particular for the coil-coating process.

5. Substrates stove-lacquered in the coil-coating process with single-component polyurethane stoving lacquers obtainable using blocked isocyanate crosslinking agents according to Claims 1 and 2.

## Revendications

1. Polyisocyanates réticulants bloqués, stables à la conservation, non cristallisables en solution, consistant en produits de réaction de
A1) 30 à 70 équivalents % d'un composant polyisocyanate à une teneur en NCO de 12 à 24 % en poids, consistant en polyisocyanates pour vernis à groupes allophanate, biuret, isocyanurate, iminooxadiazinedioine, uret-dione et/ou uréthanne, à base de diisocyanates aliphatiques linéaires, éventuellement ramifiés et
A2) 30 à 70 équivalents % d'un composant polyisocyanate à une teneur en NCO de 8 à 15 % en poids, consistant en polyisocyanates pour vernis à groupes allophanate, biuret, isocyanurate, uret-dione et/ou uréthanne, à base de diisocyanates cycloaliphatiques, la proportion totale des deux composants polyisocyanates A1 + A2 représentant 100 équivalents %, avec
B) 85 à 100 équivalents %, par rapport aux groupes isocyanate des composants A1 + A2, d'une amine aliphatique secondaire linéaire bouillant de 60 à 110°C et
C) jusqu'à 15 équivalents %, par rapport aux groupes isocyanate des composants A1 + A2, de dérivés de l'hydrazine, chimiquement combinables, de formule (I) 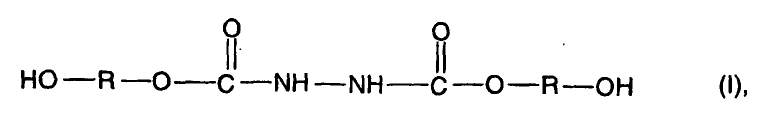 dans laquelle
R représente un radical hydrocarboné saturé en C₂-C₅, et contenant en mélange
E) jusqu'à 5,0 % en poids, par rapport aux composants A1, A2 et C, d'amines possédant le motif de structure de formule (II)

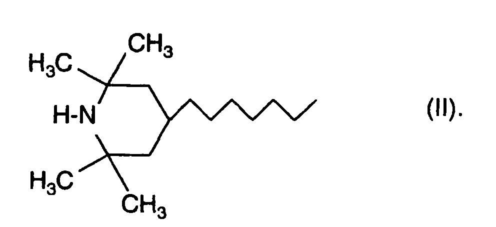

2. Polyisocyanates réticulants bloqués selon la revendication 1, **caractérisés en ce que** le composant B) consiste en la diisopropylamine.

3. Procédé pour la préparation de ces polyisocyanates bloqués dans des solvants pour vernis, **caractérisé en ce que** l'on part des composants polyisocyanate A1 + A2 dans une partie du solvant total, on fait réagir avec l'amine aliphatique secondaire bouillant de 60 à 110°C B) et le cas échéant ensuite avec le composant stabilisant C) jusqu'à consommation des groupes NCO existants, après quoi on ajoute et on mélange le composant stabilisant D) avec le reste du solvant.

4. Utilisation des isocyanates réticulants bloqués selon la revendication 1 en tant qu'agents réticulants dans des peintures à base de polyuréthannes à un seul composant et à cuire au four, prévues en particulier pour l'application sur des solénoïdes.

5. Substrats peints et cuits au four à l'aide de peintures à base de polyuréthannes à un seul composant et à cuire au four, obtenues elles-mêmes avec utilisation des isocyanates réticulants bloqués selon les revendications 1 et 2, appliquées sur des solénoïdes.
